# EUROPEAN PATENT SPECIFICATION

(11) **EP 1 608 422 B1**
(45) Date of publication and mention of the grant of the patent: **23.01.2013**
(21) Application number: 03800352.1
(22) Date of filing: 19.12.2003
(51) Int. Cl.: A61M 25/01

(54) **COMBINED LONG RAIL/SHORT RAIL IVUS CATHETER**
KOMBINIERTER KURZ FÜHRUNGSGLEIS/LANG FÜHRUNGSGLEIS IVUS KATHETER
CATHETER IVUS A RAIL LONG/ RAIL COURT COMBINES

(30) Priority: 28.03.2003 US 401467
(43) Date of publication of application: 28.12.2005
(73) Proprietor: Boston Scientific Limited, St. Michael, Barbados, West Indies (BB)
(72) Inventor: WASICEK, Lawrence, San Jose, CA 95123 (US); GASCON, Almira, Milpitas, CA 95035 (US)
(74) Representative: Golkowsky, Stefan
(86) International application number: PCT/US2003/041616
(87) International publication number: WO 2004/093963

(56) References cited:
- EP-A- 0 388 112
- US-A- 5 531 700
- US-A1- 2003 050 661
- US-B1- 6 605 062

## Description

### FIELD OF THE INVENTION

The field of the invention relates generally to catheter systems and, more particularly, to intravascular catheter assemblies comprising both long rail and short rail design features in one catheter. Such a catheter is disclosed in the US 553 1700.

### BACKGROUND OF THE INVENTION

Catheter imaging technology has long been recognized for its potential use in medical applications that involve visualizing the structure and conditions of a body. Catheters are commonly employed to help diagnose and treat medical conditions by allowing access to remote and otherwise unreachable locations within a body. For example, catheter imaging technology may be used to locate anatomy, position diagnostic and therapeutic medical devices, and monitor surgery and surgical results.

These procedures are typically performed using imaging and treatment catheters that are inserted percutaneously into the body and into an accessible vessel of the vascular system at a site remote from the vessel or organ to be diagnosed and/or treated, such as the femoral artery. The catheter is then advanced through the vessels of the vascular system to the region of the body to be treated. The catheter may include an imaging device such as an ultrasound imaging device that is used to locate and diagnose a diseased portion of the body, such as a stenosed region of an artery. The catheter may also be provided with a therapeutic device, such as those used for performing interventional techniques including balloon angioplasty, laser ablation, atherectomy, and the like. Catheters also commonly are used for the placement of grafts, stents, stent-grafts, etc., for opening up and/or preventing closure of diseased or damaged vessels.

Catheters having ultrasound imaging and/or therapeutic capabilities are generally known. For example, U.S. Patent No. 5,313,949, issued to Yock, , describes an intravascular ultrasound imaging catheter having an atherectomy cutting device. In general, there are two predominant techniques used to position the therapeutic catheter at the region of interest within the body. The first technique simply involves directly inserting the catheter into a vessel and advancing the catheter through the branches of the vascular system by pushing and steering the catheter to enter a desired branch as the catheter is moved forward. The use of this technique typically requires that the catheter be equipped with an extremely flexible guidewire at its distal tip that can be aimed in different directions by rotating the catheter or by actuating a steering mechanism.

The second technique utilizes a separate guidewire that is first positioned within the vascular system such that a distal end of the guidewire extends beyond the region of interest. The guidewire is routed into position by inserting it into a vessel and advancing it through the vascular system by pushing and steering the guidewire similar to the method previously described for a catheter. Once the guidewire is in place, the therapeutic and/or imaging catheter is routed over the guidewire to the region of interest while holding the guidewire fixed in place. The catheter being inserted includes a guidewire lumen that is sized to receive the guidewire.

The use of a guidewire provides several advantages. Routing a catheter or guidewire through a circuitous path of the complex network of blood vessels to a region of interest can be a tedious and time consuming task. Placement of the guidewire is made even more difficult with increasing vessel occlusion that may occur in the later stages of vascular disease. In addition, many catheter procedures require the use of several different catheters. For instance, an imaging catheter may be initially inserted to precisely locate and diagnose a diseased region. Then, the imaging catheter may be removed and a therapeutic catheter, such as a balloon angioplasty catheter, may be inserted. Additional therapeutic or imaging catheters may be employed as necessary. Accordingly the successive insertion and removal of each of these catheters, called catheter "exchanges," is required because there is only enough space within the vessels to rout a single catheter at a time. Hence, with the use of a guidewire, the tedious and time-consuming task of routing a device to the region of interest need only be done once. Then, the much easier procedure of routing catheters over the guidewire to the region of interest may be performed as many times as the desired therapy dictates.

Traditional catheter systems utilized a guidewire lumen that spanned the entire length of the catheter. These systems, referred to as "over-the-wire" systems, require the whole length of the catheter to be inserted over the guidewire during an exchange. Exchange of a catheter in such a system generally requires a very long guidewire or a guidewire extension. The process may be time consuming and tedious.

In order to overcome the disadvantages of the over-the-wire method, rapid exchange systems have been developed. Rapid exchange systems are generally preferred over conventional over-the-wire systems because they do not require an extended guidewire. In contrast to over-the-wire systems, a rapid exchange catheter interacts with the guidewire for a relatively short distance which allows for easier exchange of the catheter. There are two main forms of rapid exchange systems: (1) long rail (or long lumen) rapid exchange or (2) short rail (or monorail) rapid exchange. Current systems that embody a rapid exchange system generally include either a long rail or a short rail system.

A catheter that is configured to operate in long rail rapid exchange mode generally has an aperture located within about 15 to 40 cm from the distal tip that is configured to receive a guidewire. The guidewire is threaded through the distal tip of the catheter along a long rail lumen and exits out of the long rail guidewire aperture. The relatively long interaction of the catheter and guidewire as compared to short rail operation increases the trackability or pushability of the catheter. In general, the catheter will also comprise a common lumen that extends from the proximal region that is configured to house a working element. The common lumen and the guidewire lumen intersect within the distal region to form a distal common lumen. In long rail mode, the guidewire may be retracted into the guidewire lumen during operation of the working element to eliminate possible interference. After operation of the working element, the working element may be retracted proximally and the guidewire readvanced distally.

Other catheters are designed to have a short rail or monorail. Typically, a monorail catheter includes an axial opening that is located within 10 cm from the distal tip. A short rail lumen then extends distally from the opening towards the distal tip. The guidewire can be inserted into the distal tip and exit the catheter via the short rail opening. Although the short interaction of the guidewire and the monorail catheter reduces the trackability of the catheter, the working element of the catheter may be operated with the guidewire in place because the common lumen is separate from the short rail lumen.

U.S. Patent No. 5,531,700, issued to Moore et al. (the "Moore" patent) discloses a catheter comprising two guidewire ports in a distal region allowing for long rail rapid exchange and short rail rapid exchange. However, the Moore patent does not include separate distal tips for the short rail and long rail lumens. Therefore, the proximal end of the guidewire cannot be selectively advanced in the long rail lumen or short rail lumen without entering a common lumen.

It would thus be desirable to provide the improved catheter described below that allows for both long lumen and short lumen rapid exchange.

### SUMMARY OF THE INVENTION

The improved catheter body as further disclosed in claim 1 can be introduced over a movable guidewire which has been previously positioned at a desired location with the vascular system. Depending on the clinical need, the physician can choose whether to use the long rail or short rail mode.

In one example embodiment, the improved catheter has a proximal region comprising a common lumen configured to house a working element such as a transducer. The common lumen may extend into a proximal portion of the distal region. The distal region preferably comprises long rail and short rail axial openings or apertures that are each configured to receive a guidewire. For example, the long rail guidewire aperture is preferably located within about 15 to 40 cm from the distal end of the catheter and is in communication with a long rail guidewire lumen. Within the proximal portion of the distal region, the common lumen and the long rail guidewire lumen are separated by a first membrane in this embodiment. In this embodiment, the distal portion also comprises a short rail lumen that is in communication with the short rail guidewire aperture. The short rail guidewire aperture is preferably within about 1 to 10 cm from the distal end of the catheter. The short rail lumen is configured to receive a guidewire and terminates at a short rail distal tip.

In one example embodiment of a method of using the example improved catheter in long rail rapid exchange mode, the user would direct a proximal end of a guidewire into a long rail distal tip. The guidewire would then be advanced through the distal long rail lumen, distal common lumen and long rail guidewire lumen. The guidewire can then be further advanced proximally in relation to the catheter such that the proximal end of the guidewire exits out of the catheter via the long rail guidewire aperture. During operation of a working element, the guidewire may be retracted into the long rail lumen and the working element advanced into the distal common lumen. Following operation of the working element, the working element may be retracted and the guidewire readvanced.

To operate the improved catheter in an example method of a short rail mode, the user would insert the proximal end of the guidewire into the short rail distal tip. The guidewire can then be advanced through the short rail guidewire lumen and exit the catheter via the short rail guidewire aperture. During operation of the working element in short rail mode, the guidewire can remain in place because the short rail lumen does not intersect with the distal common lumen.

Other systems, methods, features and advantages of the invention will be or will become apparent to one with skill in the art upon examination of the following figures and detailed description. It is intended that all such additional systems, methods, features and advantages be included within this description, be within the scope of the invention, and be protected by the accompanying claims.

### BRIEF DESCRIPTION OF THE DRAWINGS

The details of the invention, both as to its structure and operation, may be gleaned in part by study of the accompanying figures, in which like reference numerals refer to like parts. The components in the figures are not necessarily to scale, emphasis instead being placed upon illustrating the principles of the invention. Moreover, all illustrations are intended to convey concepts, where relative sizes, shapes and other detailed attributes may be illustrated schematically rather than literally or precisely.

FIG. 1 shows an example embodiment of an improved combined long/rail/short rail catheter.

FIG. 2 shows the distal region of an example embodiment of an improved combined long/rail/short rail catheter.

FIG. 3A illustrates a cross-section of the catheter of FIG. 2 along the A-A axis.

FIG. 3B illustrates a cross-section of the catheter of FIG. 2 along the B-B axis.

FIG. 3C illustrates a cross-section of the catheter of FIG. 2 along the C-C axis.

FIG. 3D illustrates a cross-section of the catheter of FIG. 2 along the D-D axis.

FIG. 4 shows the distal region of an example embodiment of an improved combined long rail/short rail catheter during the start of operation in long rail mode.

FIG. 5 shows the distal region of an example embodiment of an improved combined long rail/short rail catheter during operation in long rail mode.

FIG. 6 illustrates the distal region of an example embodiment of an improved combined long rail/short rail catheter during operation of a working element in long rail mode.

FIG. 7 illustrates the distal region of an example embodiment of an improved combined long rail/short rail catheter during the start of operation in short rail mode.

FIG. 8 shows the distal region of an example embodiment of an improved combined long rail/short rail catheter during the operation of a working element in short rail mode.

### DESCRIPTION OF THE PREFERRED EMBODIMENTS

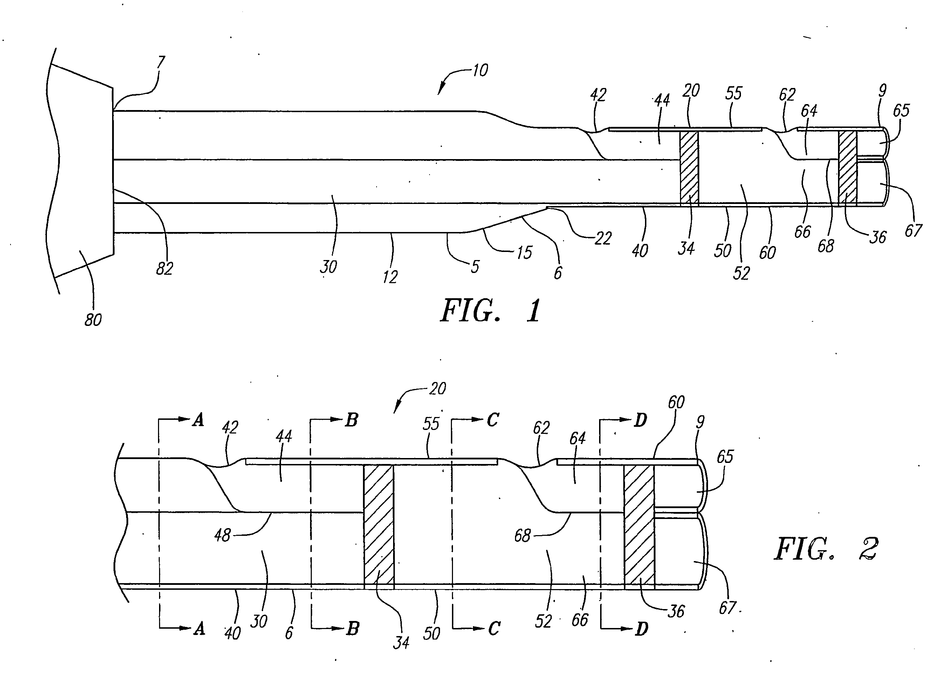

FIG. 1 illustrates an example embodiment of an improved combined long rail/short rail catheter 10 which comprises an elongate catheter body 5 having a proximal end 7 and a distal end 9. The catheter body 5 will include at least two regions, with a distal region 20 extending from the distal end 9 of the catheter body 5 to a location spaced proximally from the distal end 9 and a proximal region 12 extending proximally from the proximal end 22 of the distal region 20.

The catheter body 5 may be made any suitable material or combination of materials including Pebax 70A, Tecoflex, polyethylene, nylon, hypo-tube, natural rubber, silicone rubber, polyvinylchloride, polyurethanes, polyesters, polytetrafluorothylene (PTFE), and thermoplastic polymers. The catheter body 5 may be formed as a composite having a reinforcement material incorporated within the catheter body 5 in order to enhance strength, flexibility, and toughness. Suitable enforcement layers include wire mesh layers and the like.

The catheter body 5 generally will have a circular cross-sectional configuration. Alternatively, the cross-section of the catheter body 5 may vary depending on the method of manufacturing and desired use of the catheter 10. For example, the cross-section of the catheter body 5 may have an oval cross-section.

The length of the catheter body 5 may range depending on the application of the catheter 10. In general, the length of the catheter body 5 will be in the range of 40 to 400 cm. Preferably, the length of the catheter body 5 is in the range of 110-250 cm and most preferably in the range of 110-175 cm. The distal region 20 of the catheter body 5 will typically have a length of about 1 cm to 40 cm, more typically being in the range of about 2 cm to 30 cm.

The distal region 20 of the catheter body 5 preferably has a smaller diameter than the proximal region. The catheter body 5 may comprise a tapered region 15 disposed between the proximal region 12 and the distal region 20 that tapers downward from the proximal region 12 to the distal region 20. The diameter of the proximal region 12 of the catheter body 5 may vary depending on the method of manufacturing and the intended use of the catheter 10. The proximal region 12 of the catheter body 5 may have a diameter of, e.g. 0,508-3,048 mm (0.020-0.120 inches) and the distal region 20 may have a diameter of, e.g 0,254-3,048 mm (0.01 to 0.12 inches) (4 to 6 French). The diameter of each region need not be constant and may be subject to some variation across the length of the region.

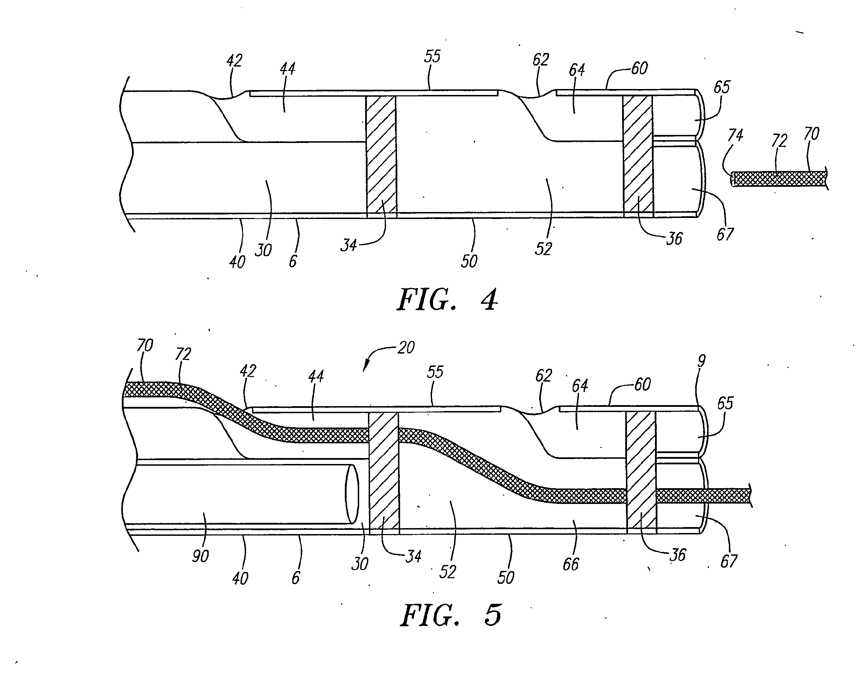

The improved combined long rail/short rail catheter is adapted to be operated with a guidewire 70 (FIG. 4). The guidewire 70 may comprise a guidewire body 72 in the form of a flexible, elongate tubular member. The guidewire body 72 may be formed of any material known in the art including nitinol hypotube, metal alloys, composite materials, plastics, braided polyimide, polyethylene, peek braids, stainless steel, or other superelastic materials.

The length of the guidewire 70 may vary depending on the application. In a preferred embodiment, the length of the guidewire 70 is between 30 cm and 300 cm. The catheter 10 may be configured to use several different diameters of guidewires 70. For example, the guidewire 70 may have a diameter of 0,254, 0,3556, 0,4572 or 0,889 mm (.010, .014, .018, or .035 inches.) In a preferred embodiment, the diameter of the guidewire 70 is substantially .014 inches. Typically, the diameter of the guidewire 70 is uniform.

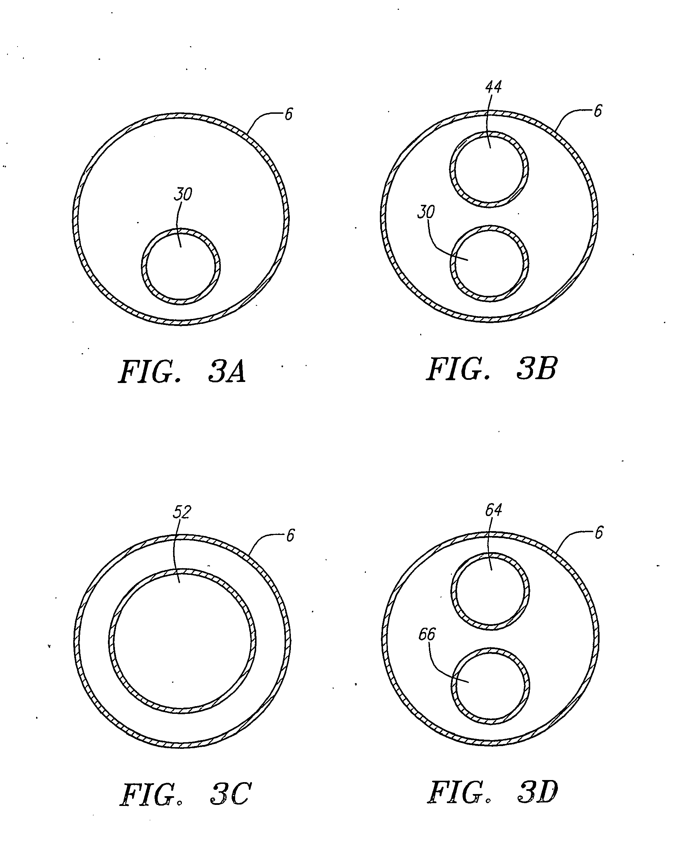

Referring to FIG. 1, the proximal region 12 of the catheter body 5 preferably has a common lumen 30 extending therethrough. The common lumen 30 is configured to receive a working element 90 attached to the distal end 82 of a drive shaft 80. The working element 90 may comprise a diagnostic or therapeutic element. The working element 90 preferably comprises an ultrasonic transducer. Alternatively, other working elements 90 may be provided depending on the needs of the patient such as light-based imaging devices, other imaging devices, cutting elements, abrasive elements, and other diagnostic and/or therapeutic elements as known in the art. In a preferred embodiment, the common lumen 30 comprises substantially smooth cylindrical walls that facilitate movement of the working element 90 along the common lumen 30 (see, e.g., FIG. 3A).

Referring to FIG. 2, the distal region may comprise at least three region. The primary region 40 of the distal region 20 is located adjacent to the proximal region 12. Therefore, the primary region 40 is the most proximate region of the three distal regions. The primary region 40 may comprise two lumens and a long rail guidewire aperture 42. The two lumens in the primary region 40 include the common lumen 30 which extends from the proximal region 12 and a long rail guidewire lumen 44, as further illustrated in FIG. 3B. An intermediate region, or secondary region 50, extends distally from the primary region 40 and comprises a single lumen. The common lumen 30 and the long rail guidewire lumen 44 connect in the secondary region 50 to form a distal common lumen 52, as illustrated in FIG. 3C. Finally, a tertiary region 60 is located in the most distal portion of the distal region 20. The tertiary region 60 comprises two lumens and a short rail guidewire aperture 62. The two lumens that comprise the tertiary region include a short rail guidewire lumen 64 and a distal long rail lumen 66, as further illustrated in FIG. 3D.

Returning to FIG. 2, the primary region 40 of the distal region 20 comprises a long rail guidewire aperture 42, a long rail guidewire lumen 44 and a distal portion of the common lumen 30. The primary region 40 preferably defines the long rail guidewire aperture 42. The long rail guidewire aperture 42 is preferably located within about 15 to 40 cm of the distal end 9. In a most preferred embodiment, the long rail guidewire aperture 42 is located within 15 to 25 cm of the distal end 9. The long rail guidewire aperture 42 may be a recessed portion of the catheter wall 6 which defines an axial opening that is configured to allow a guidewire 70 to pass through the long rail guidewire aperture 42. Preferably, the diameter of the long rail guidewire aperture 42 is approximately 0,0254 (0.001) to about 0,127 mm (0.005 inches) greater than the diameter of the guidewire 70. The long rail guidewire aperture 42 allows the catheter 10 to be used in a long lumen rapid exchange mode as is described further below.

The long rail guidewire lumen 44 extends distally from the long rail guidewire aperture 42. The length of the long rail guidewire lumen 44 preferably is between about 1 to 15 cm. The diameter of the long rail guidewire lumen 44 is sufficient to allow the guidewire 70 to slidably move within the lumen. The long rail guidewire lumen 44 and the common lumen 30 are separated by a first membrane 48.

The long rail guidewire lumen 44 and common lumen 30 extend distally to form a distal common lumen 52 in the secondary region 50 of the distal region 20. The distal common lumen 52 is preferably configured to receive either a working element 90 or a guidewire 70. Alternatively, the distal common lumen 52 may be configured to receive both a working element 90 and a guidewire 70. In order to extend the working element 90 distally past the primary region 40 and into the secondary region 50, the guidewire 70 generally will be retracted into the long rail guidewire lumen 44. Once the guidewire 70 has been displaced into the long rail guidewire lumen 44, the working element 90 can be moved distally into the distal common lumen 52. If the user wishes to reposition the guidewire 70 in long lumen rapid exchange mode after operation of the working element 90, the user may proximally retract the working element 90 into the common lumen 30. Then the user may extend the guidewire 70 from the long rail guidewire lumen 44 distally.

In a preferred embodiment, the secondary region 50 further comprises an imaging window 55. For a catheter having an acoustic transducer, The imaging window 55 is configured to facilitate use of the transducer and should be sonolucent. The intersection between the primary region 40 and the secondary region 50 of the distal region 20 may include a first radioopaque marker band 34. The first radioopaque marker band 34 may be comprised of any suitable material, including, but not limited to gold, tungsten, or platinum. Preferably, the first radioopaque marker band 34 is located approximately 12 to 40 cm from the distal end 9 of the catheter 10. As discussed further below, the first radioopaque marker band 34 may be used to assist a user in biasing a proximal end 74 of the guidewire 70 into the long rail guidewire lumen 44 during catheter exchange when operated in the long lumen rapid exchange mode.

In this example embodiment, the tertiary region 60 of the distal region 20 comprises the short rail guidewire lumen 64, a short rail guidewire aperture 62, the distal long rail lumen 66, and two distal tips 65, 67. In a further embodiment, the tertiary region 60 further comprises a second radioopaque marker band 36.

The tertiary region 60 of the distal region 20 defines a short rail guidewire aperture 62. The short rail guidewire aperture 62 is preferably located within about 1 to 10 cm of the distal end 9. More preferably, the short rail guidewire aperture 62 is located approximately 5 cm from the distal end 9. The short rail guidewire aperture 62 may be a recessed portion of the catheter wall 6 which defines an opening that is configured to allow a guidewire 70 to pass through the short rail guidewire aperture 62 and into the short rail guidewire lumen 64. Preferably, the diameter of the short rail guidewire aperture 62 is approximately 0,0254 to 0,127 mm (0.001 to about 0.005 inches) greater than the diameter of the guidewire 70. The short rail guidewire aperture 62 allows the catheter 10 to be used in a short lumen rapid exchange, or monorail, mode.

The short rail guidewire lumen 64 extends distally from the short rail guidewire aperture 62 to a short rail distal tip 65. The short rail guidewire lumen 64 is configured to receive a guidewire 70. The diameter of the short rail guidewire lumen 64 should be sufficient to accommodate the guidewire 70 and to allow the guidewire 70 to slidably move within the lumen.

The short rail distal tip 65 is configured to receive the proximal end 74 of a guidewire 70. In a preferred embodiment, the short rail distal tip 65 comprises a soft tip and may be made from any suitable material such as Pebax.

The distal common lumen 52 extends from the secondary region 50 into the tertiary region 60 to form a distal long rail lumen 66. The short rail guidewire lumen 64 and the distal long rail lumen 66 may be separated by a second membrane 68. The distal long rail lumen 66 can be used as a flush lumen. In contrast to systems with an axial flush aperture, the distal region 20 can be configured to allow for flushing out of the distal tip 67 via the distal long rail lumen 66. Therefore, when a flushing fluid is utilized, the fluid travels along the vessel. This eliminates trauma to the wall of the vessel that can be caused during flushing via an axial opening. The improved catheter provides two lumens at the distal tip of the catheter to allow the use of a working element while a guidewire is in place and to allow a guidewire to remain secured during flushing. Unlike the Moore system, the improved catheter permits flushing of the catheter and vessel while the guidewire is extended.

The distal long rail lumen 66 extends distally to a long rail distal tip 67. The long rail distal tip 67 is configured to receive the proximal end 74 of a guidewire 70. In a preferred embodiment, the long rail distal tip 67 comprises a material similar to the short rail distal tip 65 such as Pebax. The long rail distal tip 67 and short rail distal tip 65 are preferably soft tips in order to facilitate movement of the distal end 9 of the catheter 10 within the vasculature. The diameter of the long rail distal tip 67 maybe substantially equal to the diameter of the short rail distal tip 65. Alternatively, the diameter of the long rail distal tip 67 may be greater than the diameter of the short rail distal tip 65.

In a preferred embodiment, the tertiary region 60 of the distal region tapers slightly downward near the distal end 9 of the catheter 10. Therefore the diameter of the distal end 9 is preferably smaller than the diameter of the primary region 40 and the secondary region 50 of the distal region 20.

A second radioopaque marker band 36 may be provided within the tertiary region 60 of the distal region 20. The second radioopaque marker band 36 may be comprised of any suitable material, including but not limited to gold, tungsten, or platinum. Preferably, the second radioopaque marker band 36 is located within about 1 to 10 cm from the short rail distal tip 65 and most preferably is in the range of about 1 to 4 cm from the short rail distal tip 65. The second radioopaque marker band 36 is configured to assist the user in directing the guidewire 70 through either the short rail guidewire lumen 64 or the distal long rail lumen 66.

In order to operate the catheter 10 in long rail or long lumen rapid exchange mode, the user would guide a proximal end 74 of the guidewire 70 into the long rail distal tip 67 as shown in FIG. 4. The catheter 10 may then be moved distally in relation to the guidewire 70. The guidewire 70 would then move proximally through the distal long rail lumen 66 and into the distal common lumen 52. The guidewire 70 can then be positioned such that the proximal end 74 of the guidewire 70 is directed into the long rail guidewire lumen 44 instead of the common lumen 30. The guidewire may be biased to enter the long rail guidewire lumen 44 by the user. The user can detect when the proximal end 74 of the guidewire 70 has entered the primary region 40 because of the first radioopaque marker band 34 located at the intersection of the primary region 40 and the secondary region 50 as is known in the art. As the catheter 10 is moved further distally in relation to the guidewire 70, the guidewire 70 will exit the catheter 10 via the long rail guidewire aperture 42.

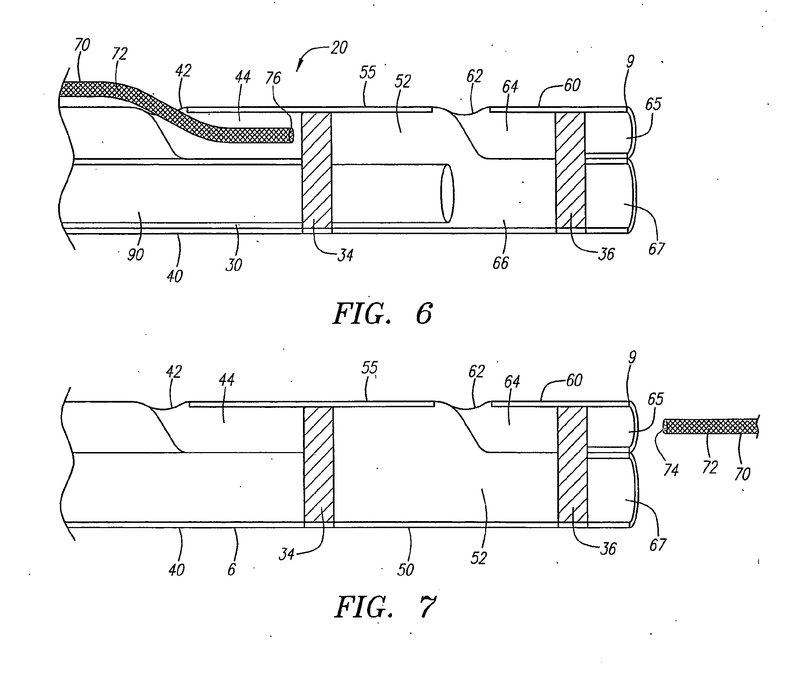

As shown in FIGs. 5 and 6, in order to operate a working element 90, the guidewire 70 may be moved distally from its engagement with a preselected region of a vessel such that the distal end 76 of the guidewire 70 is within the long rail guidewire lumen 44. The working element 90, which had been housed in the common lumen 30, may then be extended distally into the distal common lumen 52, as shown in FIG. 6. After operation of the working element 90, the working element 90 can be retracted into the common lumen 30 and the guidewire 70 moved distally to the preselected region or another region of interest.

If the user wishes to provide a flushing fluid during the procedure, the working element 90 may be fully retracted. The flushing fluid may then be deposited within the common lumen 30. The flushing fluid will then advance distally and exit the catheter 10 via the long rail distal tip 67. As stated above, the long rail distal tip 67 allows the flushing fluid to travel down the vessel in contrast to conventional flushing systems which axially release the flushing fluid.

Use of the catheter 10 in long rail mode increases the trackability of the process as compared to operation in short lumen rapid exchange mode. The increased interaction of the guidewire 70 and the catheter 10 as compared to short rail operation reduces the likelihood of bucking of the distal region 20. This increases the pushability of the catheter 10 and allows the user to more easily position the distal region 20 within a preselected region.

Additionally, if the guidewire 70 is inserted through the long rail guidewire lumen 44, the guidewire 70 may be retracted during operation of the working element 90. This allows a diagnostic working element such as a transducer to operate without potential interference or impairment from the guidewire 70.

However, sometimes operation of the catheter 10 in long rail mode may make interchanging of the catheter 10 more difficult as compared to short rail mode. As shown in FIG. 7, in order to operate the catheter 10 in short rail mode, a user would guide the proximal end 74 of the guidewire 70 into the short rail distal tip 65. The catheter may then be moved distally in relation to the guidewire 70 and the guidewire 70 would be moved proximally through the short rail guidewire lumen 64. The proximal end 74 of the guidewire 70 will then exit the catheter 10 via the short rail guidewire aperture 62. Thus, as discussed above, the proximal end 74 of the guidewire 70 may be alternately inserted into the long rail distal tip 67 and enter the long rail guidewire lumen 44 when a long rail or long lumen rapid exchange mode is desired.

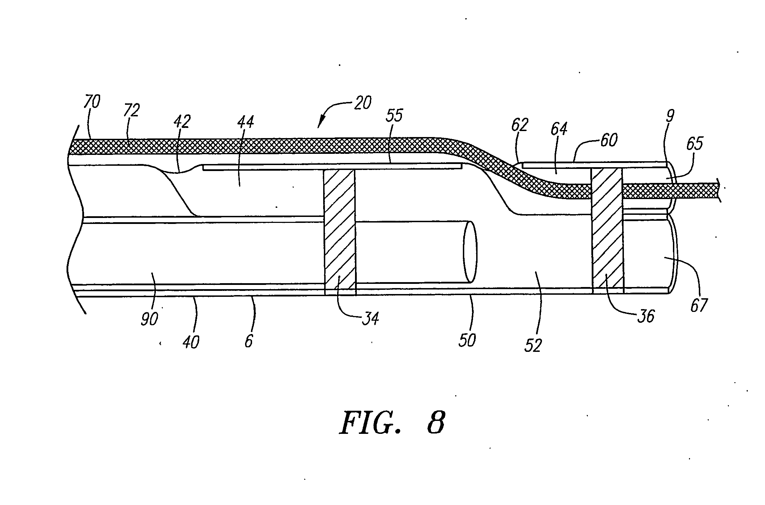

As shown in FIG. 8, use of the short rail guidewire lumen 64 allows a guidewire 70 to remain in place during operation of the working element 90. As discussed above, if the system is used with the guidewire 70 inserted into the long rail guidewire lumen 44, the guidewire 70 cannot extend past the distal end 9 of the catheter 10 when a working element 90 is in use. Instead, the guidewire 70 must be retracted into the long rail guidewire lumen 44. Therefore, use of the system in the short rail format allows the system to remain secured to a pre-selected position during use of a working element 90.

In addition, the relatively short distance of interaction between the short rail guidewire lumen 64 and the guidewire 70 allows a catheter 10 to be exchanged more easily than during long rail usage.

Because using the catheter 10 in a short lumen or monorail format may reduce the trackability of the catheter along the guidewire 70 as compared to the long rail mode, the improved combined long rail/short rail catheter permits the user to use either mode as desired.

In the foregoing specification, the invention has been described with reference to specific embodiments thereof. It will, however, be evident that various modifications and changes may be made thereto without departing from the scope of the invention. For example, the reader is to understand that the specific ordering and combination of process actions shown in the process flow diagrams described herein is merely illustrative, unless otherwise stated, and the invention can be performed using different or additional process actions, or a different combination or ordering of process actions. As another example, each feature of one embodiment can be mixed and matched with other features shown in other embodiments. As yet another example, any kind of imaging technique may be employed, such as ultrasound, light-based (e.g., OCT or OCDR), MRI, etc. Features and processes known to those of ordinary skill may similarly be incorporated as desired. Additionally and obviously, features may be added or subtracted as desired. Accordingly, the invention is not to be restricted except in light of the attached claims and their equivalents.

## Claims

1. A catheter (10) having a proximal region (12) and a distal region (20), comprising:
a long rail distal tip (67) being configured to receive a guidewire;
a long rail guidewire aperture (42) disposed through an outer surface of the distal region (20), the long rail guidewire aperture (42) being configured to receive a guidewire;
a long rail guidewire lumen (44) disposed within the distal region, the long rail guidewire lumen (44) being in communication with the long rail guidewire aperture (42) and the long rail distal tip (67);
a distal long rail lumens (66) in communication with the long rail guidewire lumen (44) and extending to the long rail distal tip (67);
a short rail distal tip (65) being configured to receive a guidewire;
a short rail guidewire aperture (62) disposed through an outer surface of the distal region (20), the short rail guidewire aperture (62) being configured to receive a guidewire; and
a short rail guidewire lumen (64) disposed within the distal region (20), the short rail guidewire lumen (64) being in communication with the short rail guidewire aperture (62) and the short rail distal tip (65),
wherein the short rail guidewire lumen (64) and the distal long rail lumen (66) are separated by a second membrane (68).

2. The catheter (10) of claim 1, further comprising a common lumen (30) disposed within the proximal region (12), wherein the common lumen (30) is configured to receive a working element (90).

3. The catheter (10) of claim 2, wherein the common lumen (30) extends into a proximal portion of the distal region (20).

4. The catheter (10) of claim 2, wherein the common lumen (30) extends into an intermediate portion of the distal region as a distal common lumen (52), the distal common lumen (52) being connected to and in communication with the common lumen (30) and the long rail guidewire lumen (44).

5. The catheter (10) of claim 4, wherein the distal common lumen (52) is configured to receive a working element (90) or a guidewire.

6. The catheter (10) of claim 1, wherein the long rail guidewire aperture (42) is located within 15 to 40 cm of the long rail distal tip (67).

7. The catheter (10) of claim 1, wherein the short rail guidewire is located within 1 to 10 cm of the short rail distal tip.

8. The catheter (10) of claim 1, further comprising a first radiopaque marker band (34), the first radiopaque marker band (34) being located at or near an overlap of the long rail guidewire lumen (44) and the common lumen (30).

9. The catheter (10) of claim 1, further comprising a second radiopaque marker band (36), the second radiopaque marker band (36) being located at or near an overlap of the short rail guidewire lumen (64) and the common lumen (30).

10. The catheter (10) of claim 1, further comprising an imaging window (55) being disposed within an intermediate portion of the distal region.

11. The catheter (11) of claim 1, wherein the short rail guidewire aperture (62) is located distally of the long rail guidewire aperture (42).

12. The catheter (10) of claim 2, further comprising a first membrane (48) between the long rail guidewire lumen (44) and the common lumen (30),

13. The catheter (10) of either claim 2 or claim 12, further comprising the second membrane (68) between the short rail guidewire lumen (64) and the common lumen (30).

14. The catheter (10) of claim 1, wherein the distal tips (65, 67) are soft.

15. The catheter (10) of claim 1, further comprising a working element (90).

16. The catheter (10) of claim 15, wherein the working element (90) includes one of an imaging device, a stent, an atherectomy device or a balloon.

17. The catheter (10) of claim 16, wherein the imaging device includes an ultrasound transducer or a light-based imager.

## Patentansprüche

1. Katheter (10) mit einem proximalen Bereich (12) und einem distalen Bereich (20), welcher aufweist:
eine distale Langschienenspitze (67), die zum Empfangen eines Führungsdrahts ausgebildet ist;
eine Langschienen-Führungsdrahtöffnung (42), die durch eine äußere Oberfläche des distalen Bereichs (20) angeordnet ist, welche Langschienen-Führungsdrahtöffnung (42) zum Empfangen eines Führungsdrahts ausgebildet ist;
ein Langschienen-Führungsdrahtlumen (44), das innerhalb des distalen Bereichs angeordnet ist, welches Langschienen-Führungsdrahtlumen (44) in Kommunikation mit der Langschienen-Führungsdrahtöffnung (42) und der distalen Langschienenspitze (67) ist;
ein distales Langschienenlumen (66) in Kommunikation mit dem Langschienen-Führungsdrahtlumen (44), das sich zu der distalen Langschienenspitze (67) erstreckt;
eine distale Kurzschienenspitze (65), die zum Empfangen eines Führungsdrahts ausgebildet ist;
eine Kurzschienen-Führungsdrahtöffnung (62), die durch eine äußere Oberfläche des distalen Bereichs (20) angeordnet ist, welche Kurzschienen-Führungsdrahtöffnung (62) zum Empfangen eines Führungsdrahts ausgebildet ist; und
ein Kurzschienen-Führungsdrahtlumen (64), das innerhalb des distalen Bereichs (20) angeordnet ist, welches Kurzschienen-Führungsdrahtlumen (64) in Kommunikation mit der Kurzschienen-Führungsdrahtöffnung (62) und der distalen Kurzschienenspitze (65) ist,
wobei das Kurzschienen-Führungsdrahtlumen (64) und das distale Langschienenlumen (66) durch eine zweite Membran (68) getrennt sind.

2. Katheter (10) nach Anspruch 1, weiterhin aufweisend ein gemeinsames Lumen (30), das sich innerhalb des proximalen Bereichs (12) befindet, wobei das gemeinsame Lumen (30) zum Empfang eines Arbeitselements (90) ausgebildet ist.

3. Katheter (10) nach Anspruch 2, bei dem das gemeinsame Lumen (30) sich in einen proximalen Teil des distalen Bereichs (20) erstreckt.

4. Katheter (10) nach Anspruch 2, bei dem das gemeinsame Lumen (30) sich in einen Zwischenteil des distalen Bereichs als ein distales gemeinsames Lumen (52) erstreckt, wobei das distale gemeinsame Lumen (52) mit dem gemeinsamen Lumen (30) und dem Langschienen-Führungsdrahtlumen (44) verbunden und in Kommunikation ist.

5. Katheter (10) nach Anspruch 4, bei dem das distale gemeinsame Lumen (52) zum Empfangen eines Arbeitselements (90) oder eines Führungsdrahts ausgebildet ist.

6. Katheter (10) nach Anspruch 1, bei dem die Langschienen-Führungsdrahtöffnung (42) sich zwischen 15 bis 40 cm von der distalen Langschienenspitze (67) entfernt befindet.

7. Katheter (10) nach Anspruch 1, bei dem der Kurzschienen-Führungsdraht sich zwischen 1 bis 10 cm von der distalen Kurzschienenspitze entfernt befindet.

8. Katheter (10) nach Anspruch 1, weiterhin aufweisend ein erstes strahlungsundurchlässiges Markierungsband (34), welches erste strahlungsundurchlässige Markierungsband (34) sich an oder nahe einer Überlappung des Langschienen-Führungsdrahtlumens (44) und des gemeinsamen Lumens (30) befindet.

9. Katheter (10) nach Anspruch 1, weiterhin aufweisend ein zweites strahlungsundurchlässiges Markierungsband (36), welches zweite strahlungsundurchlässige Markierungsband (36) sich an oder nahe einer Überlappung des Kurzschienen-Führungsdrahtlumens (64) und des gemeinsamen Lumens (30) befindet.

10. Katheter (10) nach Anspruch 1, weiterhin aufweisend ein Abbildungsfenster (55), das sich innerhalb eines Zwischenteils des distalen Bereichs befindet.

11. Katheter (10) nach Anspruch 1, bei dem die Kurzschienen-Führungsdrahtöffnung (62) sich distal von der Langschienen-Führungsdrahtöffnung (42) befindet.

12. Katheter (10) nach Anspruch 2, weiterhin aufweisend eine erste Membran (48) zwischen dem Langschienen-Führungsdrahtlumen (44) und dem gemeinsamen Lumen (30).

13. Katheter (10) nach entweder Anspruch 2 oder Anspruch 12, weiterhin aufweisend die zweite Membran (68) zwischen dem Kurzschienen-Führungsdrahtlumen (64) und dem gemeinsamen Lumen (30).

14. Katheter (10) nach Anspruch 1, bei dem die distalen Spitzen (65, 67) weich sind.

15. Katheter (10) nach Anspruch 1, weiterhin aufweisend ein Arbeitselement (90).

16. Katheter (10) nach Anspruch 15, bei dem das Arbeitselement (90) eine(n) von einer Abbildungsvorrichtung, einem Stent, einer Atherektomievorrichtung oder einem Ballon enthält.

17. Katheter (10) nach Anspruch 16, bei dem die Abbildungsvorrichtung einen Ultraschallwandler oder eine auf Licht basierende Abbildungsvorrichtung enthält.

## Revendications

1. Cathéter (10) comportant une région proximale (12) et une région distale (20), comprenant :
une pointe distale de rail long (67) qui est configurée pour recevoir un fil de guidage ;
une ouverture de fil de guidage de rail long (42) disposée au travers d'une surface externe de la région distale (20), l'ouverture de fil de guidage de rail long (42) étant configurée pour recevoir un fil de guidage ;
une lumière de fil de guidage de rail long (44) disposée à l'intérieur de la région distale, la lumière de fil de guidage de rail long (44) étant en communication avec l'ouverture de fil de guidage de rail long (42) et la pointe distale de rail long (67) ;
une lumière de rail long distale (66) en communication avec la lumière de fil de guidage de rail long (44) et s'étendant jusqu'à l'extrémité distale de rail long (67) ;
une pointe distale de rail court (65) qui est configurée pour recevoir un fil de guidage ;
une ouverture de fil de guidage de rail court (62) disposée au travers d'une surface externe de la région distale (20), l'ouverture de fil de guidage de rail court (62) étant configurée pour recevoir un fil de guidage ; et
une lumière de fil de guidage de rail court (64) disposée à l'intérieur de la région distale (20), la lumière de fil de guidage de rail court (64) étant en communication avec l'ouverture de fil de guidage de rail court (62) et l'extrémité distale de rail court (65),
dans lequel la lumière de fil de guidage de rail court (64) et la lumière de rail long distale (66) sont séparées par une seconde membrane (68).

2. Cathéter (10) selon la revendication 1, comprenant en outre une lumière commune (30) disposée à l'intérieur de la région proximale (12), dans lequel la lumière commune (30) est configurée pour recevoir un élément de travail (90).

3. Cathéter (10) selon la revendication 2, dans lequel la lumière commune (30) s'étend à l'intérieur d'une partie proximale de la région distale (20).

4. Cathéter (10) selon la revendication 2, dans lequel la lumière commune (30) s'étend à l'intérieur d'une partie intermédiaire de la région distale en tant que lumière commune distale (52), la lumière commune distale (52) étant connectée avec et en combinaison avec la lumière commune (30) et la lumière de fil de guidage de rail long (44).

5. Cathéter (10) selon la revendication 4, dans lequel la lumière commune distale (52) est configurée pour recevoir un élément de travail (90) ou un fil de guidage.

6. Cathéter (10) selon la revendication 1, dans lequel l'ouverture de fil de guidage de rail long (42) est située dans 15 à 40 cm de la pointe distale de rail long (67).

7. Cathéter (10) selon la revendication 1, dans lequel le fil de guidage de rail long est située dans 1 à 10 cm de la pointe distale de rail court.

8. Cathéter (10) selon la revendication 1, comprenant en outre une première bande de marqueur radio-opaque (34), la première bande de marqueur radio-opaque (34) étant située au niveau de ou à proximité d'un chevauchement de la lumière de fil de guidage de rail long (44) et de la lumière commune (30).

9. Cathéter (10) selon la revendication 1, comprenant en outre une seconde bande de marqueur radio-opaque (36), la seconde bande de marqueur radio-opaque (36) étant située au niveau de ou à proximité d'un chevauchement de la lumière de fil de guidage de rail court (64) et de la lumière commune (30).

10. Cathéter (10) selon la revendication 1, comprenant en outre une fenêtre d'imagerie (55) qui est disposée à l'intérieur d'une partie intermédiaire de la région distale.

11. Cathéter (11) selon la revendication 1, dans lequel l'ouverture de fil de guidage de rail court (62) est positionnée de façon distale par rapport à l'ouverture de fil de guidage de rail long (42).

12. Cathéter (10) selon la revendication 2, comprenant en outre une première membrane (48) entre la lumière de fil de guidage de rail long (44) et la lumière commune (30).

13. Cathéter (10) selon soit la revendication 2, soit la revendication 12, comprenant en outre la seconde membrane (68) entre la lumière de fil de guidage de rail court (64) et la lumière commune (30).

14. Cathéter (10) selon la revendication 1, dans lequel les pointes distales (65, 67) sont douces.

15. Cathéter (10) selon la revendication 1, comprenant en outre un élément de travail (90).

16. Cathéter (10) selon la revendication 15, dans lequel l'élément de travail (90) inclut un moyen pris parmi un dispositif d'imagerie, un stent, un dispositif d'athérectomie et un ballon.

17. Cathéter (10) selon la revendication 16, dans lequel le dispositif d'imagerie inclut un transducteur à ultrasons ou un imageur basé sur lumière.
